Europäisches Patentamt

European Patent Office

Office européen des brevets

(11) Publication number: **0 075 800**

**A1**

## (12) EUROPEAN PATENT APPLICATION

(21) Application number: **82108578.4**

(22) Date of filing: **17.09.82**

(51) Int. Cl.³: **A 61 B 5/00**
**A 61 B 1/06, F 21 V 9/04**

(30) Priority: **29.09.81 IT 2421081**

(43) Date of publication of application:
**06.04.83 Bulletin 83/14**

(84) Designated Contracting States:
**AT BE CH DE FR GB IT LI LU NL SE**

(71) Applicant: **Guidetti, Valter**
**Via Rosmini, 9**
**I-20056 Trezzo Sull'adda (Milan)(IT)**

(71) Applicant: **Stucchi, Giuseppe**
**Via Brasca, 12**
**I-20056 Trezzo Sull'adda (Milan)(IT)**

(72) Inventor: **Guidetti, Valter**
**Via Rosmini, 9**
**I-20056 Trezzo Sull'adda (Milan)(IT)**

(72) Inventor: **Stucchi, Giuseppe**
**Via Brasca, 12**
**I-20056 Trezzo Sull'adda (Milan)(IT)**

(74) Representative: **Dr. Ing. A. Racheli & C.**
**Viale San Michele del Carso, 4**
**I-20144 Milano(IT)**

(54) **A process and apparatus to display rather superficial neoformations present in some parts of the body by using at least one light source.**

(57) This invention relates to a process to display rather superficial neoformations present in some parts of the body, according to which said parts are illuminated by a light beam (11, 12, 13) focussed to display zones having a different density relative to the surrounding sound tissue. The apparatus for carrying out the process is made to be portable and provides at least one light source (8) comprising, for example, a halogen lamp, which is focussed by a suitable focussing means (6) and made to pass through colour optical filters (32, 33)

FIG.1

EP 0 075 800 A1

Applicants:

VALTER GUIDETTI

Via Rosmini, 9

IT - 20056 TREZZO SULL'ADDA (Milan)

and

GIUSEPPE STUCCHI

Via Brasca, 12

IT- 20056 TREZZO SULL'ADDA (Milan

"A PROCESS AND APPARATUS TO DISPLAY RATHER SUPERFICIAL
NEOFORMATIONS PRESENT IN SOME PARTS OF THE BODY BY USING
AT LEAST ONE LIGHT SOURCE".

This invention relates to a process and apparatus to display
rather superficial neoformations present in some parts of the
body by using at least one light source accordingly handled.
The dimensions of this apparatus are reduced, so that the
apparatus is portable.

This apparatus is particularly useful for family doctors carrying out their activity with limited diagnostic means outside of large cities. To said doctors the assisted clients first apply for any sanitary problem, for example when they have palpated or seen more or less painful suspicious neoformations on the skin or in the subskin, in the mammary gland, at the level of the penis and so on.

The doctor, essentially alone, estimates the neoformations with his own hands and sight, often he seeks advice from specialists who however use bulky, sophisticated and expensive equipments.

Particularly, referring to the mammary gland test, just consider the apparatus for mammography, which uses radiological apparatuses and is therefore dangerous both to the specialist and to the patient, in addition to being sophisticated and expensive and accordingly located in very few equipped centers.

All of this involves appointments and thus time and anxiety both for the assited person and physician.

Another known type of apparatus provides the use of thermography using the different thermal gradients to display the abnormal zones. Also this apparatus is particularly sophisticated as it must detect and display temperature gradients in the order of tenths of degree.

The apparatus using light sources for examining parts of the body are at present extremely rough.

The known type of apparatuses provides a battery which is used to illuminate a cavity of the human body such as, for example, the mouth or the ear interior. In this case the light is not focussed.

Other apparatuses comprising a light beam of variable amplitude are known, said beam being directed towards the part of the body to be examined, in particular the mammary gland. This type of apparatus:

- provides the use of only one lens to collimate the light beam rays. It does not comprise however any beam focussing means towards particular zones of the parts to be examined. Therefore only insufficient information about the observed shade can be obtained and other apparatuses are necessary to know something more;

- it does not comprise any internal heat retaining layer. Therefore, it cannot operate for a long period because of the excessive heating caused by the permanently switched-on light.

Therefore, it is the object of the present invention to provide a process which enables to examine rather superficial zones of the body by using only an accordingly handled light, i.e. focussed and possibly coloured, so as to get the maximum number of information related to the benign or malignant nature of the superficial zones of different density, if compared with the surrounding tissue, and accordingly illuminated, without resorting to more expensive and dangerous apparatuses.

The apparatus to carry out said process, should be used and readily operated by the family doctor. It should be also harmless and of moderate cost.

It is an auxiliary object to use this apparatus for early diagnosis and prevention of benign or malignant nature of rather superficial neoformations, above all if related to the mammary gland, so as to enable the physician to immediately express a diagnostic orientation with some more data than those obtainable through only his sight and hands. This is particularly important for the patient from a psychological point of view.

The object has been achieved by providing a process to display rather superficial neoformations in some parts of the body, according to which a light beam is focussed by a set of lenses or focussing means, so as to detect the density differences of the abnormal tissues by the sound surrounding tissue.

The apparatus for carrying out the process provides at least one light source and a focussing means comprising at least two lenses to address a light beam towards that part of the body to be examined.

An embodiment of the invention provides that the light source is inserted in a container lined by at least one heat insulating layer suitable to retain the heat caused by said light source.

A particular solution of the apparatus provides that the light source is inserted in a container lined by at least one layer of reflective material.

Another embodiment provides that said apparatus comprises a camera for  photographing the illuminated image.

Another solution contemplates the provision of a metal net to be disposed next to the layer of reflective material.

A preferred embodiment provides that the focussing means comprise a plano-convex lens disposed between two biconvex lenses. By suitably varying the relative length between these lenses, the focus of said light source can be modified.

An improvement provides a duct for the cooling air inlet, preferably pressurized, and an air outlet duct.

A preferred solution provides at least a colour optical filter, placed at the focussing means, so as to remove particular wavelengths of the light beam.

A preferred embodiment of the invention will now be described with reference to the accompanying drawings, in which:
Fig. 1 is a view showing an embodiment of the apparatus as a whole; and
Fig. 2 is a perspective sectional view taken along lines B-B of Fig. 1.

The portable apparatus according to the invention comprises a container 2 and a focussing part 6 accomodating the set of lenses. A switch (not shown) is provided at the end 3 of the container 2.

The base for the lamp 8 is mounted at this end and provided with its clamp with several insulating layers. In this embodiment the lamp 8 is a halogen lamp. It has power of 100 W and is usually sold with a parabola end for directing the light towards the opposite side relative to the base. The focussing means is provided at the other end 7. It comprises a plano-convex lens 11 interposed between two biconvex lenses 12 and 13. For focussing, the relative length between the lenses 11 and 12 is modified, as well as the length between the lenses 11 and 13, and 12 and 13. In this embodiment, the plano-convex lens 11 is of the type sf+16,00, while the biconvex lenses 12 and 13 are of the type sf+10,00.

However, the selection for the leus diopters would depend on the user's requirements and field to be illuminated.

Downstream to lenses 12 and 13 colour filters 32 and 33, for example manufactured by AMBICO INC, are placed. They can have different colours. It is clear in fact that, according to the different colours of the illuminated zones having different densities, it is possible to get information about the kind of the neo-formation, since the colour of the incident light is modified, and then also the shade zones colour up differently. In this embodiment skin coloured and violet coloured filters have been used. The colour choice is however made by the doctor according to his needs and objects.

The end 6a of the focussing part 6 is provided with a sloping end for adaptation thereof to the various parts of the body to be illuminated. Optionally, a camera 10 is provided which is suitable to expose on the film the image which is illuminated by the device.

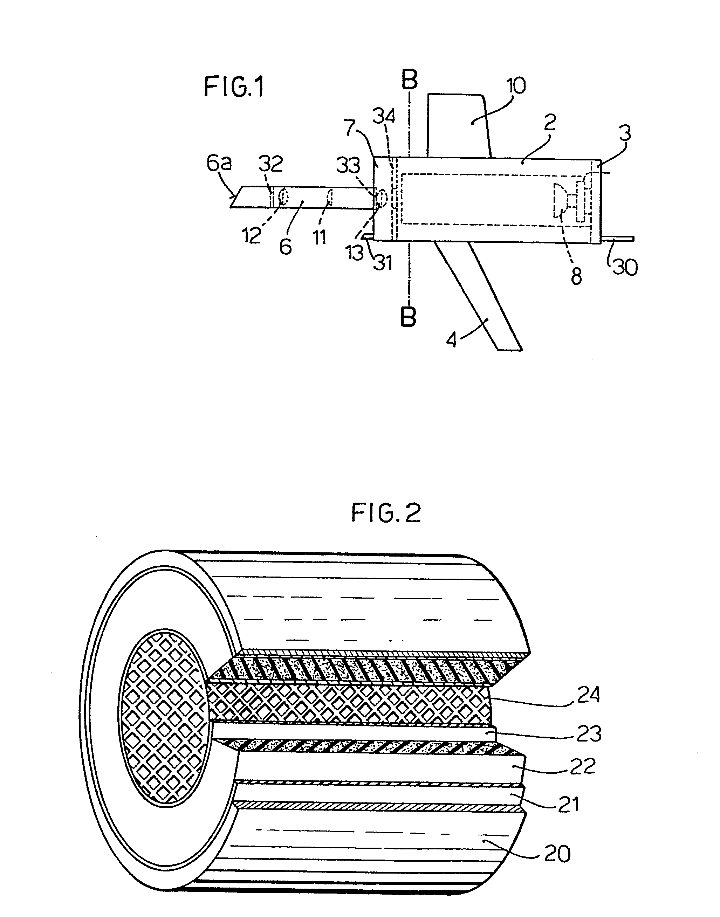

Particular care has been given to the design in order that this apparatus is not too much heating. Particularly, the container 2 has been suitably treated, that is lined or coated with several layers that are shown in Fig. 2. In this embodiment it is provided that the outer layer 20 of the container is of alluminum and 2-3 mm thick. Then a layer 21 is provided, comprising reflective material, particularly tin foil paper, then followed by a layer 22 comprising insulating material. In this embodiment the insulating material comprises glass wool and is about 1.5 cm thick. It can be used an asbestos layer,which is less thick. This solution is not shown. Next, a layer of reflective material 23 is again provided, such as still made of tin foil paper, and then a metal net 24 for avoiding the undesired reflexes of the light which is to be focussed by the focussing means.

The internal part of the device is entirely covered by asbestos, so as to reduce the external loss of heat.

The embodiment in Fig; 1 shows also an air inlet duct 30, preferably for pressurized air and a duct 31, preferably placed near the focussing means 6, for the cooling air outlet.

A brass disc 34, drilled at the set of lenses, is placed inside said apparatus. It provides a better collimation or conveyance of the light rays towards the focussing means 6.

In this embodiment the container 2 accomodating the light source and the part 6 accomodating the focussing means are distinct and cylindrically shaped. Particularly, for the container 2 the ratio diameter -to- length is 1:3, while for the focussing part the ratio diameter -to- length is 1:5. However, not necessarily the focussing part should always be outside the container, but could be provided inserted therein. To this end, technical expedients are adopted, which are well known to those skilled in the art, and which in any case do not allow the heating of the apparatus part contacting the patient's body. The whole portable apparatus is provided with a handle 4.

To conform with ENPI standards, requiring that the apparatus should operate at 12, 24 V, it is therefore necessary to provide a transformer in order that the apparatus is supplied by the mains voltage, or a specific power source anyhow making the apparatus independent of the mains supply. The wires at the outlet from the base of the lampholder and within the container are insulated.

It will clearly appear that the embodiment shown and described is only the presently preferred embodiment, while modifications can be provided to be made to the apparatus for better attaining the predetermined objects.

VALTER GUIDETTI and GIUSEPPE STUCCHI

20056 TREZZO SULL'ADDA (Milan) Italy

## C L A I M S

1.  A process to display rather superficial neoformations in some parts of the body, characterized in that a light beam is focussed by a set of lenses or focussing means (6) directed towards the parts of the body so as to detect the density differences of the abnormal tissues by the surrounding sound tissue.

2.  An apparatus for carrying out the process according to Claim 1, comprising a light source (8), movable with respect with the parts of the body to be examined, characterized by comprising at least one focussing means (6) provided by at least two lenses (11, 12, 13) to direct a light beam towards the body part to be examined.

3.  An apparatus according to Claim 2, characterized in that the light source (8) is inserted in a container (2) covered by least one heat insulating layer (22) for retaining the heat produced by said light source (8).

4.  An apparatus according to Claim 2, characterized in that the light source (8) is inserted in a container lined with at least one layer of reflective material (21, 23).

5. An apparatus according to Claim 2, characterized by the provision of a camera (10) to expose the image illuminated by the apparatus on a film.

6. An apparatus according to Claim 4, characterized by the provision of a metal net (24) to be disposed next to the layer of reflective material.

7. An apparatus according to Claim 2, characterized in that the focussing means comprises a set of lenses including a convex lens (11) interposed between two biconvex lenses (12, 13), the relative le,nght of which being selectively varied.

8. An apparatus according to Claim 2, characterized in that it comprisess a cooling air inlet duct (30) and an air outlet duct (31).

9. An apparatus according to Claim 8, characterized in that the cooling air is pressurized.

10. An apparatus according to Claim 2, characterized in that it further comprises at least one colour optical filter (32, 33) so as to remove particular wavelengths of the light beam.

FIG.1

FIG.2

European Patent
Office

**EUROPEAN SEARCH REPORT**

**0075800**
Application number

EP 82 10 8578

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | A 61 B 5/00 |
| X | US-A-4 212 306 (K.MAHMUD) *Abstract; column 2, lines 13-20; column 3, lines 10-34; column 3, line 55 - column 4, line 6; column 4, lines 25-42; column 5, lines 29-54; figures 1-6* | 1,2,10 | A 61 B 1/06 F 21 V 9/04 |
| | --- | | |
| A | US-A-4 286 602 (R.GUY) *Abstract; column 6, line 59 - column 7, line 14; column 8, lines 26-60; column 9, lines 60-67; column 10, lines 1-20; figures 1-5* | 1,2,5, 8,10 | |
| | --- | | |
| A | US-A-3 527 932 (J.J.THOMAS, B.E.NEWTON) *Abstract; column 2, lines 11-54; figure 1* | 1,2 | TECHNICAL FIELDS SEARCHED (Int. Cl. 3) |
| | --- | | |
| A | GB-A- 639 508 (PHILIPS) *Page 1, line 79 - page 2, line 2; page 2, lines 93-116; figure 2* | 1,4,7 | A 61 B 5/00 A 61 B 1/06 A 61 B 5/06 F 21 V 9/04 G 03 B 15/14 |
| | --- | | |
| A | CH-A- 196 069 (A.FUNK) *Page 2, left-hand column, lines 34-42; figure 3* | 1,8 | |
| | --- | | |
| A | US-A-2 452 450 (C.H.FREDENBURG) *Column 2, lines 11-21; figure 2* | 3 | |
| | --- -/- | | |

The present search report has been drawn up for all claims

| Place of search THE HAGUE | Date of completion of the search 05-01-1983 | Examiner RIEB D.K. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

**European Patent Office**

## EUROPEAN SEARCH REPORT

| DOCUMENTS CONSIDERED TO BE RELEVANT | | | Page 2 | |
|---|---|---|---|---|
| Category | Citation of document with indication, where appropriate, of relevant passages | Relevant to claim | CLASSIFICATION OF THE APPLICATION (Int. Cl. ³) | |
| A | EP-A-0 025 969 (OLYMPUS OPTICAL CO., LTD.) *Abstract; page 4, line 29 - page 5, line 16; page 9, line 24 - page 10, line 8; figures 1,6-8* | 1,6 | | |
| | --- | | | |
| A | EP-A-0 027 361 (OLYMPUS OPTICAL CO., LTD.) *Abstract; page 2, line 29 - page 3, line 24; figure 1* | 1,8,9 | | |
| | ----- | | | |
| | | | TECHNICAL FIELDS SEARCHED (Int. Cl. ³) | |
| | The present search report has been drawn up for all claims | | | |
| Place of search | Date of completion of the search | | Examiner | |
| THE HAGUE | 05-01-1983 | | RIEB D.K. | |